# EUROPEAN PATENT APPLICATION

(11) **EP 4 186 477 A1**
(43) Date of publication of application: **31.05.2023**
(21) Application number: 21210116.6
(22) Date of filing: 24.11.2021
(51) Int. Cl.: A61F 2/86, A61F 2/90

(54) **METHOD OF PREFORMING AN IMPLANTABLE POLYMERIC ENDOLUMINAL SUPPORT STRUCTURE**

(71) Applicant: Stentit BV, 5612 AJ Eindhoven (NL)
(72) Inventor: Sanders, Bart, Eindhoven (NL); von Basum, Golo, Eindhoven (NL); Trommelen, Maria Alida Antonia, Eindhoven (NL); Lichauco, Arturo Miguel, Eindhoven (NL)
(74) Representative: DTS Patent- und Rechtsanwälte Schnekenbühl und Partner mbB

(57) **Abstract**

The present invention relates to a method of preforming an implantable polymeric endoluminal support structure (20) for delivery and deployment into a body, the method including:
- forming a fibrillated tubular structure (10) made of polymeric fibers, said fibrillated tubular structure (10) having a first diameter, and
- crimping the fibrillated tubular structure (10) down to a second diameter, said second diameter being smaller than the first diameter.

## Description

The present invention belongs to the technical field of implantable polymeric endoluminal support structures, e.g., stents and/or scaffolds for use, e.g., in regenerative cardiovascular interventions.

In particular, the present invention relates to a method of preforming an implantable polymer stent for delivery and deployment into a body, e.g., in a bodily lumen such as a blood vessel.

US 10,813,777 B2 discloses a tubular stent which is made of a bioabsorbable network of polymeric fibers that can be rearranged upon expansion, accommodated for diameter enlargement without the need of a strut or strut pattern, and provide temporary support to a biological duct. Additionally, a stent is provided where the rearranged fibrous network, in its expanded state, can act as a scaffold for cell infiltration and promote autologous tissue formation.

In cardiovascular interventions, implantable endoluminal support structures such as stents may serve as minimally-invasively deliverable scaffolds.

For the purpose of delivery and deployment into a bodily lumen, it is known to use stents having an initial nominal configuration with a smaller diameter, which is then expanded after deployment (e.g., upon being inflated with a balloon).

Nevertheless, this known approach has the drawback that endoluminal support structures, especially polymeric stents, tend to recoil to their initial smaller nominal configuration after deployment and inflation.

As a solution, it is known to manufacture stents according to the size they should have and maintain after deployment at a target site within a bodily lumen (e.g., a blood vessel) and then crimp the stent down to smaller diameter for deployment purposes.

In particular, stents are subjected to a crimping procedure by using a crimping device that applies a radial compression that reduces the diameter of the stent.

Then, once the stent has been deployed at a desired target site into the bodily lumen, the diameter of the stent is radially expanded, e.g., by self-expansion or by inflating a balloon.

Crimping devices are known in the art and - in case of balloon expandable stents - commonly include a balloon of a catheter assembly inside the lumen to crimp the stent onto such balloon. The stent is placed at the center of the device and then crimping is performed by applying radial compression that allows to obtain a desired reduction of the diameter of the stent and to mount the stent firmly onto the balloon of the catheter assembly.

Delivery and deployment of a stent are commonly accomplished by positioning the stent around one end of a catheter, inserting said end of the catheter into a bodily lumen (e.g., a blood vessel), advancing the catheter to a target site, expanding the stent at the target site, and finally removing the catheter from the lumen (US8298466B1).

The stent must allow for crimping and expansion without incurring in any structural and / or functional damages to its structure.

Further, it is necessary that the stent maintains its size and shape after deployment at the target site, despite loads and forces acting on the same after deployment.

For instance, once deployed and expanded in a target site of a blood vessel, the stent needs to withstand the radial forces exerted by the walls of the vessel, further than the cyclic loading induced by the beating heart or movement of an artery in the body (especially in the leg or other peripherals).

US8298466B1 discloses a method of fabricating a stent having improved capability of mechanical support to walls of a bodily lumen, said method comprising: disposing a bioabsorbable polymer tube in a chamber; contacting the tube with a fluid at supercritical conditions, wherein the fluid is impregnated in the polymeric tube; adjusting conditions in the chamber so that the fluid is subcritical to form a porous structure in the tube; and reducing or preventing radial expansion of the tube during formation of the porous structure.

US2017348124A1 discloses a method of uniform crimping and expansion of a medical device such as a scaffold. A catheter balloon is pressurized at relatively high pressures prior to crimping to allow pre-arranged folds of the balloon to be substantially removed. Then, when the balloon reaches an inflated state, the scaffold is crimped to the balloon until the scaffold diameter has been reduced in size to about 50% (or more). At this point, the balloon pressure is relieved to avoid damage to the balloon and to achieve a small crossing profile.

US2017252191 (A1) discloses a method for crimping a polymeric coated stent on a balloon catheter. The stent can be either a metallic stent or a bioabsorbable polymeric stent having a coating that comprises a polymer and a rapamycin derivative drug. Crimping is conducted in an environment having a relative humidity of 45% to 55% and at a temperature greater than 25°, where humidity level is maintained until the stent is considered crimped and compressed on the balloon by a crimping apparatus.

However, there is still room for improvements.

In particular, there is a need for enhancing the capability of an endoluminal support structure such as a stent to maintain its size and shape after deployment, and to improve adhesion of said structure to the walls of a bodily lumen, e.g., a blood vessel, thereby preventing the risk of unwanted displacement after deployment.

This object is achieved by the provision of a method of preforming an implantable endoluminal support structure according to claim 1.

Accordingly, a method of preforming an implantable polymeric endoluminal support structure for delivery and deployment into a body is provided, wherein the method comprises the following steps:
- forming a fibrillated tubular structure of polymeric fibers, the fibrillated tubular structure having with a first diameter, and
- crimping the fibrillated tubular structure down to a second diameter, said second diameter being smaller than the first diameter.

In particular, the present invention provides a method of preforming an implantable endoluminal support structure, for delivery and deployment into a body, in particular a bodily lumen such as a blood vessel.

The endoluminal support structure can be, for instance, a polymeric fibrillated stent.

The tubular structure is preferably made of a network of polymeric fibers in the micrometric or nanometric range. Reference is made especially to US 10,813,777 B2.

In particular, the tubular structure can be a stent that is obtained through electrospinning.

The invention is based on the basic idea that, by providing an endoluminal support structure made of polymeric fibers, it is possible to obtain an improved implantable structure (e.g., a polymer stent) having an enhanced capability of maintaining its size and shape once deployed, regardless of loads and forces acting on it once in site. Said structure further shows an improved capability of adhering to the walls of a bodily lumen, e.g., a blood vessel, into which it is deployed.

In particular, the main idea is that the support structure, such as a stent structure, is crimped down from a larger first diameter to a second smaller diameter. Such stent structures as e.g. described in US 10,813,777 B2 have a shape memory, i.e. they (can) return to their initial shape upon certain temperature and environmental factors such as the presence of water or other substances. Thereby, it shall be prevented a situation where, after implantation, the stent structure tends to return to it is initial configuration (which it had before being deployed and inflated so as to be attached to the inner vessel wall), which could also mean that the stent structure returns to a smaller diameter and therefore does no longer maintain its position within the vessel.

According to the basic idea of the invention, the stent is initially provided with its nominal length and diameter, then crimped down to a smaller diameter, which is then used for the deployment. While being deployed, the stent will return to its nominal diameter, i.e. its regular length and diameter. With this size and configuration, it is placed at the implantation site within the blood vessel. Further influence of temperature, water uptake and other influencing factors, which might cause that the stent structure returns to its nominal state and configuration cannot cause a shrinking of the stent anymore as the stent is in the implanted and deployed state already in its nominal state.

The inventors witnessed with fully polymeric fibrillated structures (stents) the issue of intrinsic stent recoil. Here, the stent (without being subject to the method according to the invention) was first made in small diameter configuration, inflated, and exposed to elevated physiological temperature (fever conditions). Then, stents went back to their nominal small diameter configuration. There appeared to be a short of "shape memory effect" in the stent, wanting to go back to nominal configuration when exposed to elevated physiological temperatures in an aqueous environment. This led to the problem that the stent became smaller after removing the balloon, for which the stent itself was providing a stenosis inside the artery. As a solution, the inventors realized that the stents should be provided in the size how they should be at the implantation site (the new nominal state). Then, the stent will be crimped and then when they will be inflated and exposed to elevated temperature, the stents want again to go back to their initial state, but this time the large diameter. In this way the stent will not shrink at the implantation site. This has already been seen and proven in the lab. This way, the structure can be influenced such that in any case the stent structure will return to the pre-crimping form, in case that the factors which cause a shape-memory effect apply. It also helps that no unwanted irreversible structure change or material change happens during the process.

Preferably, crimping is performed at or around glass transition temperature (T_{g}) of said polymeric fibers. In this temperature range the shape memory of the stent structure and its material can be set in a very good manner.

More preferably crimping is performed at room temperature.

For instance, crimping may be performed at a temperature ranging from 34° to 43°.

In particular, crimping may be preferably performed at about 37° (i.e., approximately the body temperature of a healthy human subject).

Temperature range at so-called normal physiological conditions are preferable for performing the method. This way, it is ensured that the material is treated with the method in temperature ranges, which will also apply after implantation.

Advantageously, crimping may be performed in an environment having humidity level of about 20% to 100%.

For example, crimping can be performed in an aqueous solution. However, it is also possible to perform the step of crimping at dry conditions without the presence of an aqueous solution.

For instance, crimping may include performing crimping of a wetted fibrillated tubular structure.

Adding an aqueous solution or performing the method at a certain humidity can influence to lower the glass transition temperature (T_{g}) of the material and thereby further enhance the structural features of the stent so obtained in terms of improved capability to crimp the stent.

In particular, said aqueous solution can be water. In trials, normal drinkable water has been used. It is also possible to use isotonic water, which matches with the physiological conditions of the solutes of a human, saline, or phosphate buffered saline.

The method may further include allowing the fibrillated tubular structure to rest in a crimped state after crimping, before release. This allows relaxing of the fibers from the crimping.

In case crimping is performed in an aqueous solution (e.g., water), said step of allowing the fibrillated tubular structure to rest in a crimped state can be performed while being immersed in said aqueous solution.

Further, in case crimping is performed in an aqueous solution at relatively high temperatures (e.g., ranging from 34° to 43°, preferably around 37°), the method may include:
- removing the fibrillated tubular structure from said aqueous solution, and
- letting the fibrillated tubular structure to cool down below glass-transition temperature (T_{g}) while being maintained in the crimped state.

Then, the crimping load can be released and the fibrillated tubular structure is dried.

Alternatively, the fibrillated tubular structure can be dried before the crimping load is released.

For instance, drying of the electrospun tubular structure (either before or after releasing the crimping load) can be performed by using compressed air.

Support structures such a stents usually include a porous structure made of a polymer material that is hydrophobic.

Accordingly, when exposed to water or bodily fluids such as blood, air remains trapped in said porous structure and inhibits water or bodily fluids from penetrating.

The inventors have found that wetting of the fibrillated tubular structure prior to crimping is beneficial since it eases reorganization of the fibrous mesh and makes inflation simpler. Further, wetting of the structure of the material will enhance and ease cell infiltration into the structure, it will prevent embolisms of air being trapped in the structure and released after implantation, will support coating or can be used as carrier for pharmaceutical agents. Further, the wetting will help that the overall stent structure has a homogenous stent expansion characteristic.

It has been surprisingly found by the inventors that the stent structure made of polymeric fibers as described herein in this disclosure has a tendency to go back to the initial state and have this shape memory. This is inter alia related to the difference between "dry" glass transition temperature (T_{g}) and "wet" glass transition temperature (T_{g}). The used polymers have a T_{g} above body temperature. This means, that at body temperature the polymer is in a glass state, meaning the material is stiff, brittle, and strong. Since small fibers will be mainly used for the stent structure, water may have an intense effect on the hydration of the fibers used in the stent structures. This may lead to a rapid drop in the T_{g} of the polymer as water molecules start interfering with the polymer chains within the fibers. As it progresses, the T_{g} drops to reach physiological body temperature. Here, the polymer transits from a glass transition towards a rubbery state. This will enable the polymer chains to freely move again. What happens next is that because of this, the build up stresses and strains induced by deformation due to the crimping can dissipate by which it will go back to its initial strain and stress free (or more optimal) starting configuration.

This also explains why the stent structures will remain crimped as long as they are dry and exposed to temperatures below its Dry T_{g}. But as soon as the constructs become humid, the T_{g} drops and regains its "shape memory" effect.

To this end, the method may further include subjecting the fibrillated tubular structure to high centrifugal force while being immersed in an aqueous solution, preferably water prior to crimping, so that air is removed from the network of electrospun polymeric fibers and replaced with the aqueous solution.

In particular, said aqueous solution can be water.

The high centrifugal force can be applied for approximately 30 seconds at, for example, 15000 x g, relative centrifugal force (RCF).

The crimping is performed while the tubular structure is already mounted around an expandable balloon of a catheter assembly. This way, the process of manufacturing a ready-to-implant and ready-to-deployment product is significantly enhanced. For placing the stent structure on the balloon anyhow a kind of crimping or the like is needed. So, there is only one crimping step which integrates the dimension setting and the placement on the balloon of the balloon catheter. So the crimping method can be used to mount the stent on a balloon catheter device. In any case it is important that the stent is firmly crimped on the balloon. Therefore, after crimping, the stent may not (gradually) expand during shelf life or delivery into the body. Also, it should be firmly fixed that it withstands sufficient "dislodgement force" by pushing it through tight, rough and calcified lesions or when advancing it though introducer devices. This is achieved with the method as explained above.

The invention further provides a fibrillated tubular structure having the features of claim 15.

The fibrillated tubular structure has a first configuration before crimping and a second configuration after crimping, wherein crimping is performed through the method described above.

In particular, said fibrillated tubular structure may distinguish:
(i) in the first configuration before crimping, a first state with a first diameter of the fibrillated tubular structure, where the fibrillated network is determined by a first fiber orientation characterized by a first fiber dispersion and a first main angle difference, and a first average fiber diameter, and
(ii) in the second configuration after crimping, a second state with a second diameter of the fibrillated tubular structure, where the fibrillated network is determined by a second fiber orientation characterized by a second fiber dispersion and a second main angle difference, and a second average fiber diameter,
wherein the second diameter of the fibrillated tubular structure is smaller than the first diameter of the fibrillated tubular structure.

The fibrillated network may be arranged according to a random fiber orientation scenario.

In this case, the first fiber dispersion is smaller than the second fiber dispersion.

Alternatively, the fibrillated network may be arranged according to a controlled fiber orientation scenario.

In this case, the first main angle difference is equal to or smaller than the second main angle difference.

Alternatively, the fibrillated network may be arranged according to a combination of a controlled fiber orientation and a random fiber orientation scenario.

Alternately the fibrillated network may be composed of multiple layers of a controlled fiber orientation or a random fiber orientation or a combination of both

Advantageously, in the first configuration before crimping, the polymeric fibers forming the network are arranged according to a circumferentially aligned configuration.

The same arrangement of the polymeric fibers is then obtained once the fibrillated tubular structure is re-expanded to its original state after deployment at the target site.

This allows to enhance the load bearing capacity of the fibrillated tubular structure and benefit native-like tissue formation. In particular, cells can align along the fibers of the structure and produce a tissue component (e.g., collagen) in the same direction, resembling the native configuration.

In the first configuration for before crimping, the fibrillated tubular structure may have an inner diameter of 100 mm or less.

In the second configuration after crimping, the fibrillated tubular structure may have an increased wall thickness than in the first configuration before crimping.

Then, the wall thickness of the fibrillated tubular structure is decreased again when it is re-expanded to its original state after deployment at the target site.

Further, in the second configuration after crimping, the fibrillated tubular structure may substantially have the same length as in the first configuration before crimping.

Thus, according to the invention, wall thickness of the fibrillated tubular structure may vary between the first and second configurations, while the length of the fibrillated tubular structure remains substantially unchanged.

The material for the implantable polymer stent can be biocompatible polymer. Biocompatible polymeric fiber materials can include inter alia, but not limited to it:
- Bioresorbable polymers (such as poly lactic acid (PLA), including poly(L-lactide), poly(D-lactide), poly(D,L-lactide), as well as polyglycolide, polycaprolactone, polydioxanone, poly(trimethylene carbonate), poly(4-hydroxybutyrate), poly(ester amides) (PEA), polyurethanes, poly(trimethylene carbonate), poly(ethylene glycol), poly(vinyl alcohol), polyvinylpyrrolidone, and copolymers thereof such as poly(L-lactide/DL-lactide), poly(L-lactide/D-lactide), poly(L-lactide/glycolide), poly(L-lactide/caprolactone), poly(DL-lactide/glycolide),
- non bioresorbable materials (such as polypropylene, polyethylene, polyethylene terephthalate, polytetrafluoroethylene, polyaryletherketone, nylon, fluorinated ethylene propylene, polybutester, and silicone, or copolymers thereof),
- biological components (such as hyaluronan, collagen, gelatin, chitosan, alginate, aloe/pectin, cellulose or other biological materials originating from tissues from either autologous, allergenic or xenogenic origin),
- or a combination thereof.

Advantageously, the polymeric fibers may include: poly(L-lactide), poly(D-lactide), polyglycolide or a combination thereof in the form of a co-polymer being either poly(DL-lactide), poly(lactide-co-glycolic acid) or poly(DL-lactide-co-glycolic acid).

In particular, said crystalline or semi-crystalline polymeric material may have a glass-transition temperature (T_{g}) above physiological core body temperature.

Further details and advantages of the present invention shall now be disclosed in connection with the drawings.

It is shown in:
- **Fig. 1a**: an implantable endoluminal support structure, in particular a stent, before and after performing a crimping step according to an embodiment of the present invention;
- **Fig. 1b**: a view similar to Fig. 1a, but from a different perspective;
- **Fig. 2**: a condition where a fibrillated tubular structure is cooled down at room temperature after crimping, while a crimping load is still exerted on said structure;
- **Fig. 3a-3e**: a sequence where air is removed from the fibrillated tubular structure by applying high centrifugal force, and the fibrillated tubular structure is hydrated with an aqueous solution;
- **Fig. 4a-4f**: different views of arrangements of polymeric fibers forming the fibrillated tubular structure in a first configuration before crimping, respectively a second configuration after crimping, in an exemplary embodiment where said polymeric fibers are disposed according to a random fiber orientation scenario;

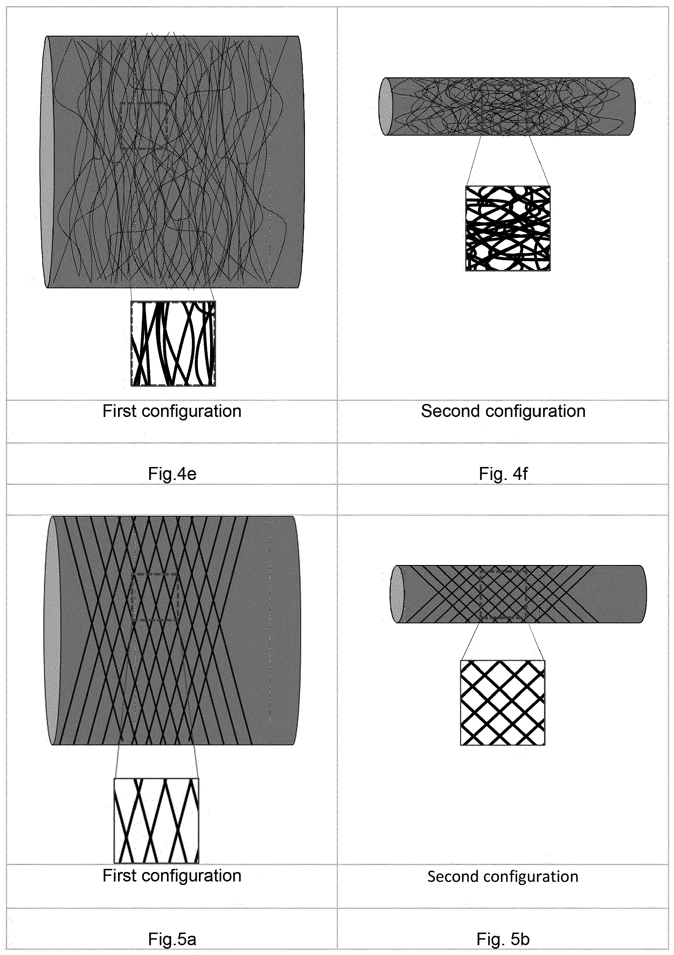

- **Fig. 5a-5b**: different views of arrangements of polymeric fibers forming the fibrillated tubular structure in the first configuration for before crimping, respectively the second configuration after crimping, in an exemplary embodiment where said polymeric fibers are disposed according to a controlled fiber orientation scenario; **Fig**. **6a-**6c an image analysis based on microscopically obtained images illustrating how main angle and dispersion of the polymeric fibers alter upon transitioning from the first configuration for before crimping to the second configuration for after crimping, in a case where the polymeric fibers are disposed according to the random fiber orientation scenario;
- **Fig. 7a-7c**: an image analysis based on microscopically obtained images illustrating how main angle and dispersion of the polymeric fibers alter upon transitioning from the first configuration before crimping to the second configuration after crimping, in a case where the polymeric fibers are disposed according to the controlled fiber orientation scenario;
- **Fig. 8**: a schematical drawing regarding the problem of the intrinsic stent recoil (prior art);
- **Fig. 9**: a schematical drawing regarding the intrinsic stent expansion according to the present invention;
- **Fig. 10**: examples of the intrinsic stent recoil as shown in Fig. 10 (upper part of Fig. 12) and the intrinsic stent expansion according to Fig. 11 (lower part of Fig. 12); and
- **Fig. 11**: a diagram regarding the polymer glass transition temperature Tg.

The present invention provides a method of preforming an implantable polymeric endoluminal support structure 20 for delivery and deployment into a body, in particular a bodily lumen such as a blood vessel.

In the present example, said endoluminal support structure 20 is a stent 20.

For example, the stent 20 may be obtained by electrospinning.

For "stent", it is intended a structure for providing structural support to a bodily lumen, e.g., a blood vessel, upon self-expansion or balloon expansion after deployment at a target site.

The method includes forming a fibrillated structure 10 made of a network of electrospun polymeric fibers, said tubular structure 10 having a first diameter. The method further includes crimping the fibrillated tubular structure 10 so obtained down to a second diameter, said second diameter being smaller than the first diameter.

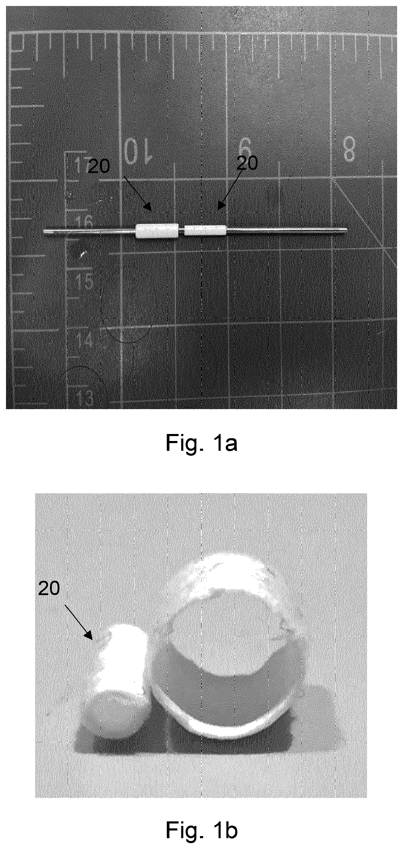

**Figs. 1a and 1b** shows the stent 20 in a first configuration for pre-deployment, respectively in a second configuration for deployment, in which the fibrillated tubular structure 10 forming the stent 20 is crimped to a smaller diameter.

The present invention allows obtaining an improved endoluminal support structure 20 such as a stent 20 having an enhanced capability of maintaining its size and shape after deployment, and which better adheres to the walls of a bodily lumen, e.g., a blood vessel once deployed, thereby preventing the occurrence unwanted displacement.

Crimping may be performed at temperatures below melt transition temperature (Tₘ) of said polymeric fibers, preferably at or around glass transition temperature (T_{g}) of said polymeric fibers, more preferably at room temperature.

In particular, crimping may be performed at a temperature ranging from 34 °C to 43 °C, preferably at about 37 °C.

Crimping may be performed in an environment having humidity level of about 20% to 100%.

In the present embodiment, crimping is performed in an aqueous solution.

In the present embodiment, said aqueous solution is water, for example water from the tap or drinkable water.

In particular, in the present embodiment, crimping is performed in said aqueous solution at a temperature ranging from 34 °C to 43 °C.

Preferably, crimping is performed in said aqueous solution at about 37 °C, i.e., at a temperature that is close to the body temperature of a healthy human.

The fibrillated tubular structure 10 is allowed to rest in a crimped state after crimping, before release.

This allows for relaxation of the polymeric fibers from the crimping.

In the present embodiment, the step of allowing the fibrillated tubular structure 10 to rest in a crimped state is performed while being immersed in said aqueous solution.

In particular, in the present embodiment, crimping is performed in said aqueous solution at a relatively high temperature.

In particular, in the present embodiment, the method further includes:
- removing the fibrillated tubular structure 10 from said aqueous solution, and
- letting the fibrillated tubular structure 10 to cool down at room temperature while being maintained in the crimped state.

For "relatively high temperature" it is intended a temperature ranging from 34 °C to 43 °C, preferably about 37° (i.e., the body temperature of a healthy human subject).

For "room temperature" it is intended a temperature of about 20°.

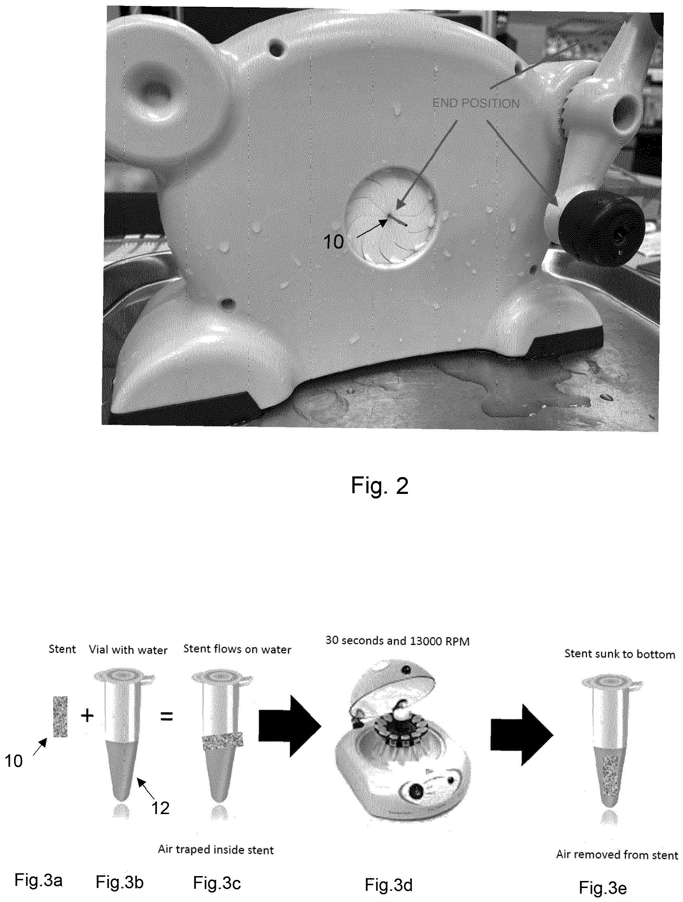

**Fig. 2** shows a condition where the fibrillated tubular structure 10 is cooled down at room temperature (e.g., about 20°) after crimping, while a crimping load is still exerted on said structure 10.

Wetting of the fibrillated tubular structure 10 before crimping can be beneficial since it eases reorganization of the fibrous mesh and makes inflation simpler.

**Figs. 3a-3e** show a sequence where air is removed from the fibrillated tubular structure 10 by applying high centrifugal force, and the fibrillated tubular structure 10 is hydrated with an aqueous solution.

In particular, in the present embodiment, the fibrillated tubular structure 10 is subjected to high centrifugal force while being immersed in an aqueous solution, preferably water before crimping, so that air is removed from the polymeric fibers and replaced with the aqueous solution.

In the shown embodiment (**Figs. 3a-3e**), the fibrillated tubular structure 10 (**Fig. 3a**) is immersed in a vial 12 that is filled with said aqueous solution (**Figs. 3b-3c**) and then subjected to high centrifugal force while being immersed in the aqueous solution (**Fig. 3d**).

For example, that said high centrifugal force can be applied for approximately 30 seconds at about 15000 x g relative centrifugal force

The fibrillated tubular structure 10 is then maintained in the vial 12 until reaching a state where it is sunk to the bottom of the vial 12, this meaning that air present in the fibers has been almost completely replaced with the aqueous solution (**Fig. 3e**).

The present invention further provides a fibrillated tubular structure 10 having a first configuration before crimping and a second configuration after crimping, where crimping is obtained by implementing the above described method.

According to the invention, said fibrillated tubular structure distinguishes:
(i) in the first configuration before crimping, a first state with a first diameter of the fibrillated tubular structure 10, where the fibrillated network is determined by a first fiber orientation characterized by a first fiber dispersion and a first main angle difference, and a first average fiber diameter, and
(ii) in the second configuration after crimping, a second state with a second diameter of the fibrillated tubular structure 10, and where the fibrillated network is determined by a second fiber orientation characterized by a second fiber dispersion and a second main angle difference, and a second average fiber diameter,
wherein the second diameter is smaller than the first diameter.

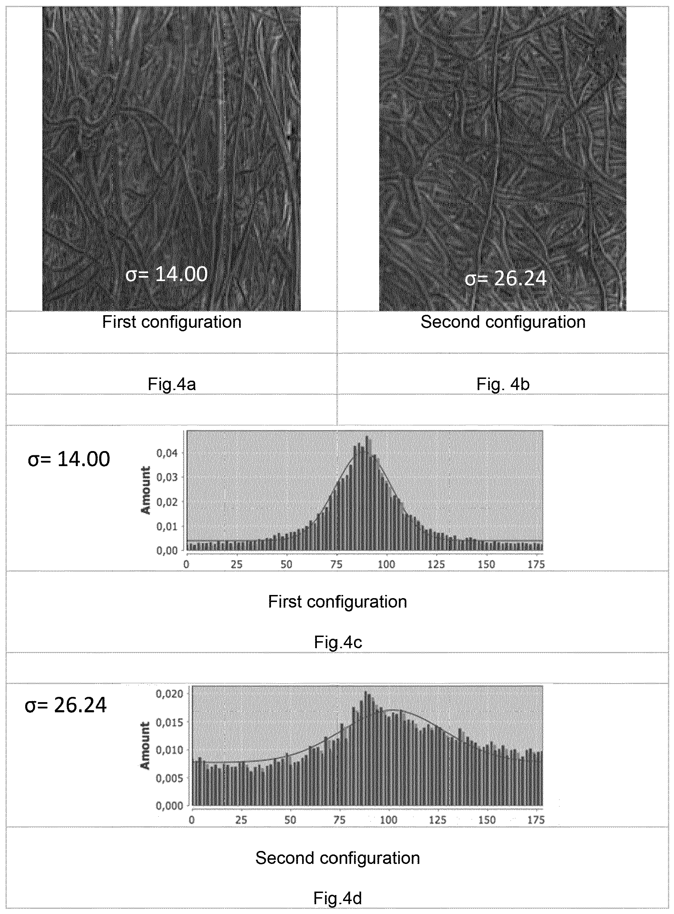

In one embodiment, the polymeric fibers may be arranged according to a random fiber orientation scenario (**Figs. 4a-4f**)**.**

**Figs. 4a-4f** show arrangements of the polymeric fibers in the first configuration for pre-deployment, respectively the second configuration for deployment, in a case where the fibers are disposed according to a random orientation.

The first fiber dispersion is smaller than the second fiber dispersion.

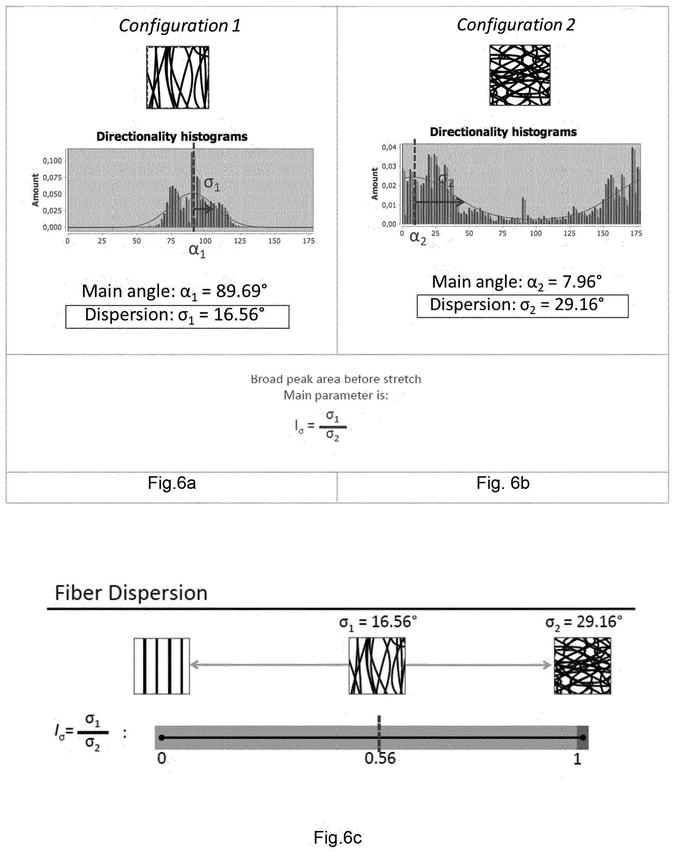

In particular, in the first configuration before crimping, the tubular structure 10 has an enlarged diameter where fibers are aligned. The directionality histogram describes a narrowed peak area surrounding one preferred orientation (**Fig. 6a**). This is captured by a lowered value of σ and a value of a which approaches 90 degrees, here intended as the circumferential direction. To prove fiber alignment, the degree of fiber dispersion is quantified by comparing σ in the first configuration before crimping (σ 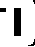 ) with respect to σ in the second configuration after crimping (o2) (**Figs. 6a-6b**).

When deployed at the target site within a bodily lumen, the stent 20 is re-expanded (either naturally or upon inflation with a balloon) to its original state where fibers are substantially aligned.

In an alternative embodiment, the polymeric fibers may be arranged according to a controlled fiber orientation scenario (**Figs. 5a-5b**)

**Figs. 5a-5b** show arrangements of the polymeric fibers in the first configuration for pre-deployment (**Fig. 5a**), respectively the second configuration for deployment **(****Fig. 5b****)**. The first main angle difference is equal to or smaller than the second main angle difference.

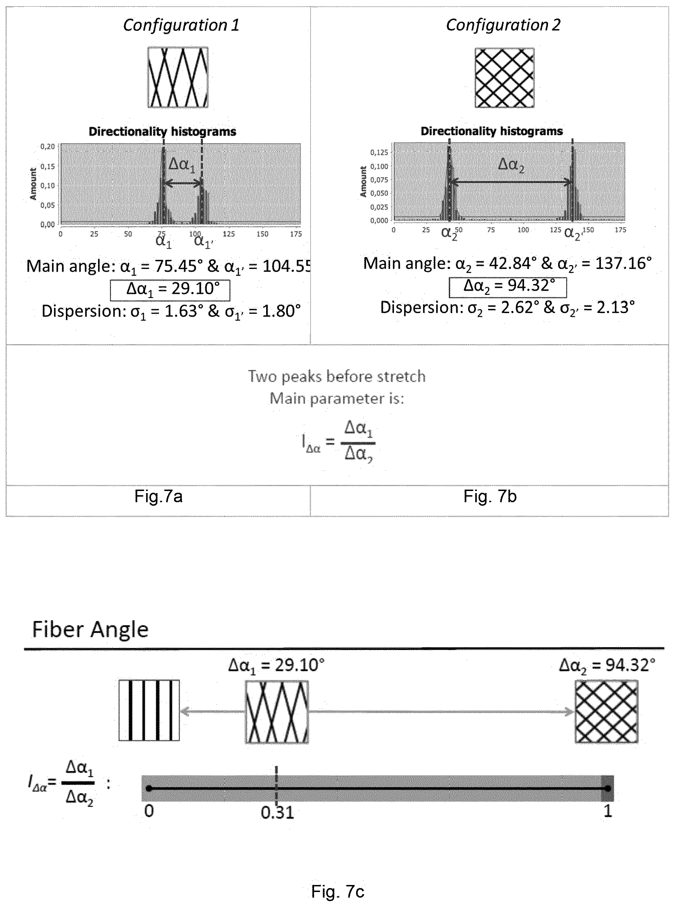

In the first configuration for pre-deployment (**Fig. 5a**), the tubular structure 10 has an enlarged diameter. The directionality histogram describes two narrow peaks, which have become closer to 90 degrees, describing circumferential alignment (**Fig. 7a**).

Advantageously, in the first configuration before crimping, the polymeric fibers may be arranged according to a circumferentially aligned configuration.

The same arrangement of the polymeric fibers is then obtained once the stent 20 is re-expanded to its original state after deployment at the target site.

In said first configuration before crimping, the fibrillated tubular structure 10 may have an inner diameter of 100 mm or less.

In the second configuration after crimping, the fibrillated tubular structure 10has an increased wall thickness than in the first configuration before crimping.

Furthermore, in the second configuration after crimping, the fibrillated tubular structure has substantially the same length as in the first configuration before crimping. This is apparent by observing the stent 20 before and after crimping, as shown in **Fig. 1a**.

That is, the wall thickness of the tubular structure 10 varies between the first and second configurations (according to a "sponge effect"), while the length of the tubular structure 10 remains substantially unchanged.

Advantageously, the polymeric fibers may include: poly(L-lactide), poly(D-lactide), polyglycolide or a combination thereof in the form of a co-polymer being either poly(DL-lactide), poly(lactide-co-glycolic acid) or poly(DL-lactide-co-glycolic acid).

The stent 20 may be mounted over the balloon of a balloon catheter.

Then, once the stent 20 is deployed at a target site within a bodily lumen, the balloon is inflated to re-expand the diameter of the stent 20 to its original configuration.

As the balloon is deflated, the stent 20 maintains its expanded configuration without shrinking.

Alternatively, the stent 20 may be self-expandable.

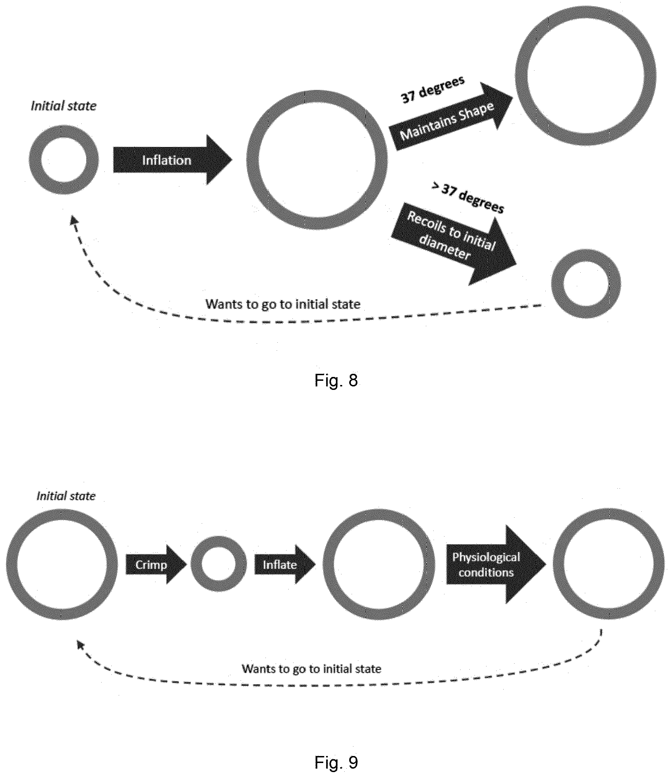

Also in this case, once re-expanded to its original configuration, the stent 20 maintains the expanded configuration without shrinking. **Fig. 8** shows a schematical drawing regarding the problem of the intrinsic stent recoil with a stent 100 according to the prior art.

Here, the stent 100 is provided as e.g. described in the prior art and made of a electrospun polymer material. Its diameter in in the initial state (cf. left part of **Fig. 8**) is smaller than in the inflated state (cf. middle left part of **Fig. 8**).

At 37 degrees the stent 100 will maintain its shape (cf. upper right part of **Fig. 8**).

In case that the temperature is above 37 degrees, then the stent 100 can show the problem of recoiling to its initial diameter, i.e. it shows a tendency to go to its initial state (cf. lower right part of **Fig. 8**).

**Fig. 9** shows a schematical drawing regarding the intrinsic stent expansion according to the present invention with a stent 20 according to the present invention.

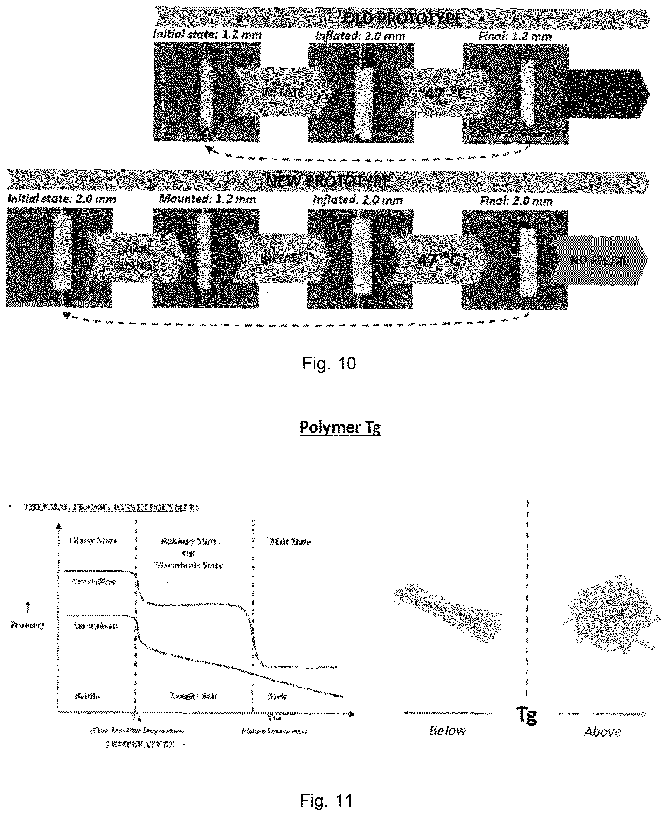

As shown in the left part of **Fig. 9****,** the stent diameter of the stent 20 is larger before the crimping, but with lower wall thickness. With the crimping, the stent 20 has a smaller diameter and an increased wall thickness when compared with the initial state (cf. middle left part of **Fig. 9**). The stent 20 then goes back to the initial diameter during deployment by means of inflation with the balloon of the balloon catheter (cf. middle right part of **Fig. 9**). It also remains in this state, which is basically the initial state of the stent 20 (cf. right part of **Fig. 10**).

**Fig. 10** shows examples of the intrinsic stent recoil as shown in **Fig. 8** (cf. upper part of **Fig. 10**). The initial state of stent 100 has a diameter of 1.2 mm. It is then inflated and has a diameter of 2.0 mm. It has been fond that at a temperature of 47 °C the stent 100 was subject to recoiling and therefore went back to a diameter of 1.2 mm.

**Fig. 10** also shows in its lower part the intrinsic stent expansion according to **Fig. 9****.** The initial state of stent 20 has a diameter of 2.0 mm. It is then crimped and reduced to a diameter of 1.2 mm. The, it is inflated and has a diameter of 2.0 mm. It has been found that at a temperature of 47 °C the stent 20 shows no recoiling and therefore stays at a diameter of 2.0 mm.

**Fig. 11** shows a diagram regarding the polymer glass transition temperature Tg. In the left part and in the diagram there the upper curve shows the behavior of a crystalline polymer and the lower curve the behavior of an amorphous polymer. As shown in the right part, below the Tg the polymer shows an oriented structure, while above Tg the polymer shows a random orientation.

### References

- 10: Fibrillated tubular structure
- 12: Vial
- 20: Implantable polymeric endoluminal support (stent)
- 100: stent (prior art)

## Claims

1. A method of preforming an implantable polymeric endoluminal support structure (20) for delivery and deployment into a body, the method including:
- forming a fibrillated tubular structure (10) made of polymeric fibers, said fibrillated tubular structure (10) having a first diameter, and
- crimping the fibrillated tubular structure (10) down to a second diameter, said second diameter being smaller than the first diameter.

2. The method of claim 1, **characterized in that** crimping is performed at a temperature below melt transition temperature (Tₘ) of said polymeric fibers, preferably at or around glass transition temperature (T_{g}) of said polymeric fibers, more preferably at room temperature.

3. The method of claim 2, **characterized in that** crimping is performed at a physiological core body temperature ranging from 34 °C to 43 °C, preferably at about 37 °C.

4. The method of any one of the preceding claims, **characterized in that** crimping is performed in an environment having humidity level of about 20% to 100%.

5. The method of claim 4, **characterized in that** crimping is performed in an aqueous solution.

6. The method of claim 5, **characterized in that** said aqueous solution is selected among:
- water,
- isotonic water;
- saline, or
- phosphate buffered saline.

7. The method of claim 4, **characterized in that** crimping includes performing crimping of a wetted fibrillated tubular structure (10).

8. The method of any one of the preceding claims, **characterized in that** it further includes allowing the fibrillated tubular structure (10) to rest in a crimped state after crimping, before release.

9. The method of claim 8, **characterized in that** said step of allowing the fibrillated tubular structure (10) to rest in a crimped state is performed while being immersed in the aqueous solution.

10. The method of any one of claims 3, 5 or 6 and 9, **characterized in that** it further includes:
- removing the electrospun fibrillated tubular structure (10) from said aqueous solution, and
- letting the fibrillated tubular structure (10) to cool down to a temperature below glass-transition temperature (T_{g}) while being maintained in the crimped state.

11. The method of any one of the preceding claims, **characterized in that** it further includes subjecting the tubular fibrillated structure (10) to high centrifugal force while being immersed in an aqueous solution, preferably water, before crimping, so that air is removed from the polymeric fibres and replaced with said aqueous solution.

12. The method of claim 11, **characterized in that** said high centrifugal force is applied for approximately 30 seconds at about 15000 x g relative centrifugal force.

13. The method of any one of the preceding claims, **characterized in that** crimping is performed to mount the fibrillated tubular structure (10) onto a balloon of a catheter assembly or to load the fibrillated tubular structure (10) into a delivery sheath.

14. The method of any one of the preceding claims, **characterized in that** the endoluminal support structure (20) is a stent (20) obtained through electrospinning.

15. A fibrillated tubular structure (10) having a first configuration before crimping and a second configuration after crimping, wherein crimping is performed through the method of any one of claims 1 to 14.

16. The fibrillated tubular structure (10) of claim 15, **characterized in that** said fibrillated tubular structure (10) distinguishes:
(i) in the first configuration before crimping, a first state with a first diameter of the fibrillated tubular structure (10), where a fibrillated network is determined by a first fiber orientation **characterized by** a first fiber dispersion and a first main angle difference, and a first average fiber diameter, and
(ii) in the second configuration after crimping, a second state with a second diameter of the fibrillated tubular structure (10), where the fibrillated network is determined by a second fiber orientation **characterized by** a second fiber dispersion and a second main angle difference, and a second average fiber diameter,
wherein the second diameter of the fibrillated tubular construct (10) is smaller than the first diameter of the fibrillated tubular construct (10).

17. The fibrillated tubular structure (10) of claim 15, **characterized in that** the fibrillated network is arranged according to a random fiber orientation scenario, wherein the first fiber dispersion is smaller than the second fiber dispersion.

18. The fibrillated tubular structure (10) of claim 15, **characterized in that** the fibrillated network is arranged according to a controlled fiber orientation scenario, wherein the first main angle difference is equal to or smaller than the second main angle difference.

19. The fibrillated tubular structure (10) of any one of claims 15 to 18, **characterized in that,** in the first configuration before crimping, the polymeric fibers forming the network are arranged according to a circumferentially aligned configuration.

20. The fibrillated tubular structure (10) of any one of claims 15 to 19, **characterized in that,** in the first configuration before crimping, said fibrillated tubular structure (10) has an inner diameter of 100 mm or less.

21. The fibrillated tubular structure (10) of any one of claims 15 to 20, **characterized in that,** said fibrillated tubular structure (10) has an increased wall thickness in the second configuration after crimping than in the first configuration before crimping.

22. The fibrillated tubular structure (10) of any one of claims 15 to 21, **characterized in that,** in the second configuration after crimping, said fibrillated tubular structure (10) has substantially the same length than in the first configuration before crimping.

23. The fibrillated tubular structure (10) of any one of claims 15 to 22, **characterized in that** the polymeric fibers include a biodegradable material, preferably a crystalline or semi-crystalline biodegradable material.

24. The fibrillated tubular structure (10) of any one of claims 15 to 23, **characterized in that** the polymeric fibers include a polymeric material selected among:
- polymer of poly(L-lactide),
- poly(D-lactide),
- poly(DL-lactide),
- polyglycolide,
- polycaprolactone, or
- a combination thereof.

25. The fibrillated tubular structure (10) of claim 24 **characterized in that** the polymeric fibers include a polymeric material comprising a co-polymer among:
- poly(L-lactide/DL-lactide),
- poly(L-lactide/D-lactide),
- poly(L-lactide/glycolide),
- poly(L-lactide/caprolactone),
- poly(DL-lactide/glycolide), or
- a combination thereof.

26. The fibrillated tubular structure (10) of claim 23 to 25, **characterized in that** said crystalline or semi-crystalline polymeric material has a glass-transition temperature (T_{g}) above physiological core body temperature of a human.
